Europäisches Patentamt

European Patent Office

Office européen des brevets

(11) Publication number: **0 267 737**
**A2**

(12) **EUROPEAN PATENT APPLICATION**

(21) Application number: **87309790.1**

(22) Date of filing: **05.11.87**

(51) Int. Cl.4: **G01L 1/16** , G01N 33/02

(30) Priority: **08.11.86 GB 8626711**

(43) Date of publication of application:
**18.05.88 Bulletin 88/20**

(84) Designated Contracting States:
**AT BE CH DE ES FR GB GR IT LI LU NL SE**

(71) Applicant: **SYRINX INNOVATIONS LIMITED**
**74 Great King Street**
**Edinburgh EH3 6QU(GB)**

(72) Inventor: **Strachan, John Scott**
**6 Marchhall Crescent**
**Edinburgh EH16 5HN(GB)**

(74) Representative: **Wotherspoon, Graham**
**Kirkland House Bonhill Alexandria**
**Dunbartonshire G83 9HT(GB)**

(54) **Apparatus for measuring firmness.**

(57) The apparatus makes use of a transducer (10) comprising a polymeric piezoelectric film (12) having electrodes (20, 22) and secured by adhesive (14) to a rigid plate (16) which in turn is mounted on a resilient, yielding support (18). The object to be assessed (26) is caused to impact on the transducer (10) to cause an electrical output from the film (12). The rigid plate (16) is chosen to have a mass which is small in relation to that of the object (26) and of a material which is non-resonant under the impact, and the support (18) is such that the film (12), the plate (16) and the object (26) move in contact during the impact. This arrangement has the result that the output signal from the film (12) represents the resonance of the object due to the impact, which can be used as a measure of the firmness of the object. The invention is particularly applicable to measuring the ripeness of fruit.

Fig. 1

## "Apparatus for measuring firmness"

This invention relates to an apparatus for measuring the degree of firmness of objects. The invention is particularly applicable to measuring the ripeness of fruit and vegetables, and will hereinafter be described in relation to this, but may also find utility with other objects such as balls for sporting use.

The degree of ripeness of fruit and vegetables (hereinafter for convenience together referred to simply as fruit) is a factor of considerable commercial importance. Various attempts have been made to develop automatic equipment for assessing ripeness in large-scale processing, for example to separate tomatoes into grades of ripeness suitable for differing uses. Most such attempts have tried to simulate human methods of judging colour or feel. However prior art approaches have not been satisfactory. Significant differences in ripeness may be accompanied by only small, subtle changes in colouration. For example, an unripe tomato is a uniform greenish colour, while a "breaker" tomato which is much riper and is usable for certain commercial purposes may be a similar colour with only a small patch of reddish hue. Assessing "feel" by applying mechanical compression to the fruit is liable to cause unacceptable damage to the fruit, and requires complex mechanical arrangements.

An object of the invention, which is defined in the appended claims, is to overcome or mitigate these problems.

Embodiments of the invention will now be described, by way of example only, with reference to the drawings, in which:

Fig. 1 is a schematic cross-section of a transducer used in the invention;

Fig. 2 is a plan view of one apparatus using a number of such transducers;

Fig. 3 is aperspective view of another apparatus using the transducers;

Figs. 4a and 4b are graphs representing the frequency spectra produced by impact between the transducer and ripe and unripe fruit; and

Fig. 5 illustrates in generalised form the transducer output voltage againsy time for ripe and unripe fruit.

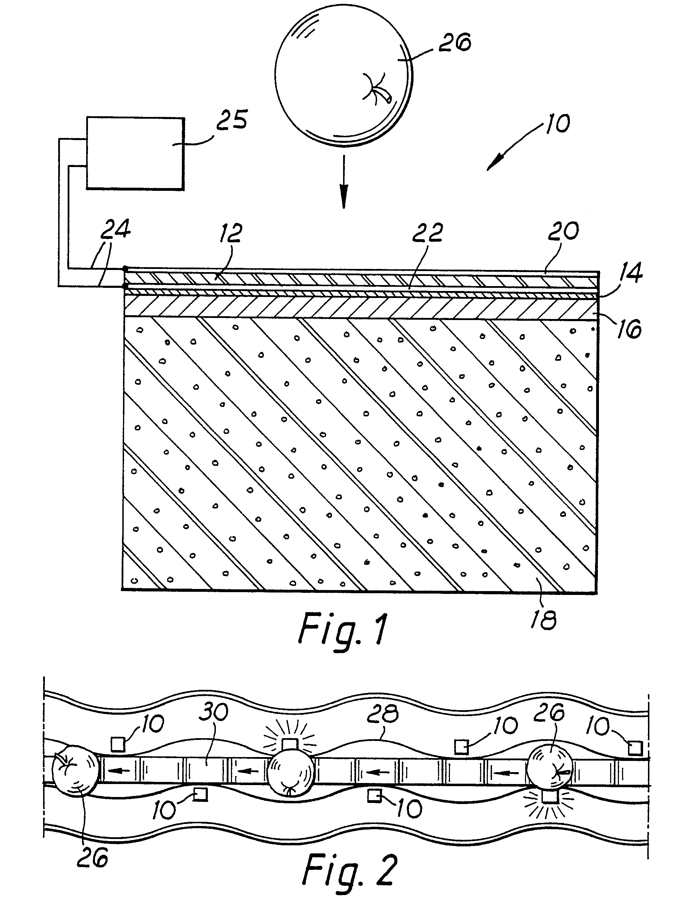

Referring to Fig. 1, the invention utilises a transducer generally designated at 10 and comprising an assembly of a polymeric piezoelectric film 12 secured by a double-sided adhesive tape 14 to a metal plate 16 which is mounted (for example by adhesive) on a relatively thick block of foam material 18. The film 12 is provided on its opposed faces with electrodes 20 and 22 (greatly exaggerated in thickness in the drawing) connected to give an output on leads 24 to an electronic circuit 25.

The film 12 is preferably of polyvinylidene fluoride (PVDF), for example KYNAR (trade mark) film by Pennwalt Corporation. A suitable film has a thickness of about 28$\mu$m and is made by melt extrusion of vinylidene fluoride homopolymer, followed by stretch orientation and electrical polarization by application of a high voltage field at controlled pressure and temperature in a manner well known per se. The electrodes 20, 22 are formed on the film 12 for example by vapour deposition of silver or by printing with silver ink, as is also well known per se. The tape 14 is suitably an acrylic transfer tape 0.010 inch thick.

In operation, a fruit 26 is caused to impact the transducer 10. Compression of the film 12 produces, owing to the piezo activity of the PVDF material, a voltage output on leads 24. The invention operates by allowing the fruit itself to resonate in response to the impact. To this end, the metal plate 16 provides a rigid member against which the film 12 is pressed during the impact, and the foam material 18 is sufficiently thick and compliant that its yielding gives a relatively long contact time during which the fruit is acoustically coupled with the film 12. It is important, because of this mode of action, that the plate 16 does not itself resonate. Annealed brass 0.75 mm thick meets this requirement; soft aluminium alloy of about the same thickness would also be suitable. For use with fruit such as tomatoes or apples, the foam 18 is suitably a light spongy foam such as polyether foam about 12 mm thick. It is also necessary that the mass of the plate 16 be small relative to that of the fruit to avoid affecting the response of the fruit. For use with apples and tomatoes, for example, the foregoing brass plate is suitable with an area 1 inch (25mm) square.

The output produced by this arrangement has a waveform and frequency spectrum which is characteristic of ripeness independently of the mass of the fruit and the speed of the impact. The use of the soft foam 18 also has the important benefit of isolating the film 12 from ambient noise, which may be severe in a mechanised sorting plant. It may be useful to cover the film 12 and electrode 20 with a thin layer of a resilient substance such as latex.

The electronic circuit 25 comprises any suitable circuitry for recovering from the transducer output information relating to the ripeness of the fruit, such information being discussed in more detail below. Given the general principles and specific examples set out herein, it will be apparent to those skilled in the art how this may be achieved and the circuitry, which does not in itslef from part of the present invention, is therefore not described in detail herein.

Fig. 2 shows the transducer 10 used in conjunction with a singulator conveyor(that is, a conveyor arranged to carry individual fruits in separate divisions defined by transverse ridges) of known type. A plurality of transducers 10 are mounted adjacent the sides of the conveyor 30, and a sinuous gully 28, or appropriately positioned cam surfaces, rolls each fruit 26 against each transducer 10 as it passes to produce the desired contact. The results for each fruit from the several transducers can be averaged before being analysed.

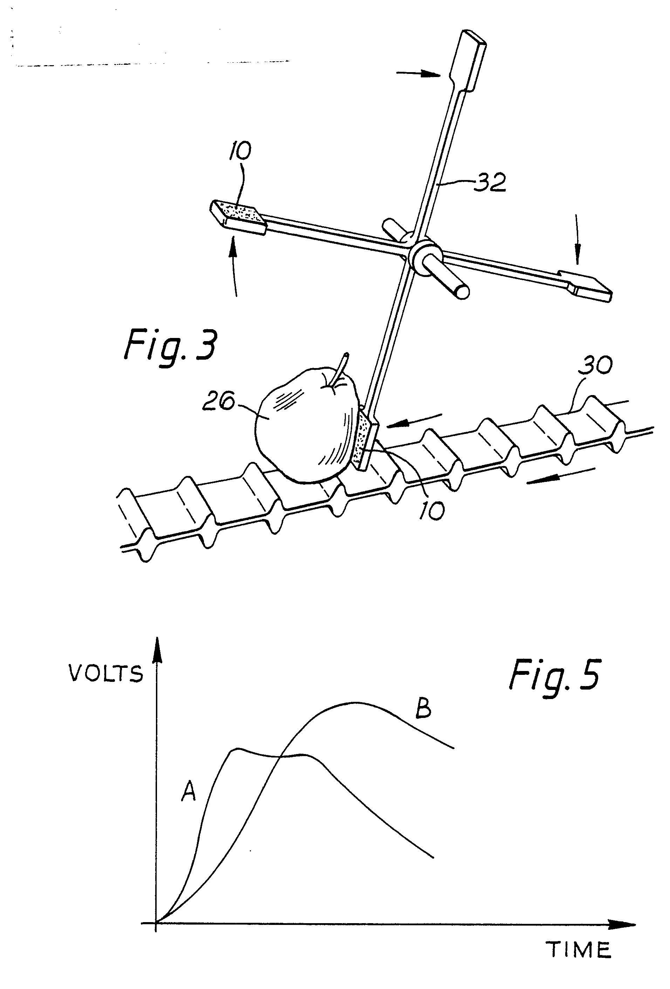

Fig. 3 shows an arrangement in which a fruit 26 on a singulator conveyor 30 is contacted by a transducer 10 mounted on a paddle device 32 which is rotated at a speed sufficiently high in relation to the speed of the conveyor to ensure that a contact occurs.

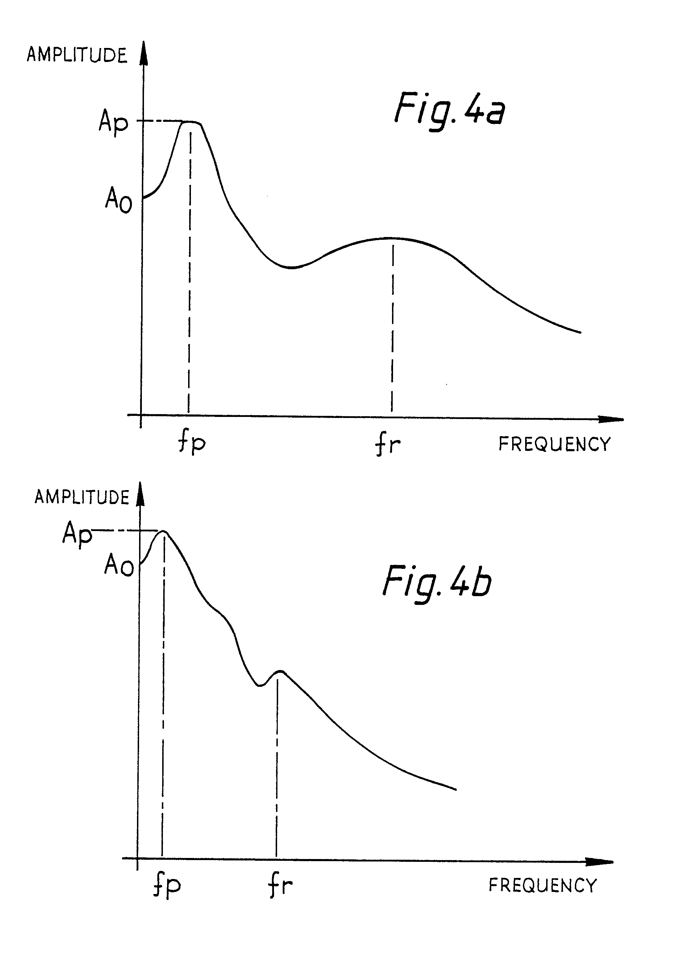

Figs. 4a and 4b show respectively the frequency spectra obtained from under-ripe and ripe tomatoes.

In these Figures:

$A_o$ = amplitude of zero Hz component

$A_p$ = amplitude of peak component

$f_p$ = frequency of peak component

$f_r$ = resonant frequency

It has been found that the less ripe the fruit the higher the frequency response of the system. Fig. 5 shows typical voltage/time outputs from the transducer for unripe fruit (curve A) and for ripe fruit (curve B). Practical measures of ripeness can thus be obtained, for example, by:

(a) measuring the rise time of the transducer output voltage,

(b) measuring the initial slope of the transducer output voltage,

(c) filtering selected frequencies from the transducer output and comparing the amplitudes of these frequencies: for example, it has been found that with peaches it is convenient to extract the 80 Hz and 800 Hz frequencies, and that the 800 Hz component is larger than the 80 Hz component with unripe fruit but vice versa with ripe fruit.

Electronic circuits for carrying out such measurements are readily available in the art and need not be described herein. It is also possible in principle to analyse the transducer output in a more sophisticated manner, for example by digitising the signal and subjecting this to computer analysis, which would make it possible to detect other factors such as weight and the presence of disease induced faults which produce characteristic frequency patterns.

## Claims

1. Apparatus for measuring the firmness of an object forming part of a given class of objects; the apparatus comprising a transducer and means for producing relative motion between the object and the transducer to produce an impact therebetween; and in which the transducer comprises:

a sheet of polymeric piezoelectric material having electrodes formed on opposed surfaces thereof,

a rigid member to which said sheet is secured, the rigid member having a mass which is small in comparison with said object and being substantially devoid of resonance when impacted by the object, and

a resilient support mounting the rigid member so as to yield and permit the rigid member to move with the object during said impact.

2. The apparatus of claim 1, in which said piezoelectric material is polyvinylidene fluoride.

3. The apparatus of claim 1, in which said rigid member is a sheet of annealed brass or soft aluminium.

4. The apparatus of claim 1, in which said resilient support is a block of soft plastics foam.

5. The apparatus of claim 1, for use with fruit, in which the transducer is about 25mm square, the rigid member is an annealed brass plate about 0.75mm thick, and the resilient support is of plastics foam about 12.5mm thick.

6. The apparatus of claim 1, in which the means for producing relative motion comprises a conveyor along which said objects are carried in spaced relation.

7. The apparatus of claim 6, in which at least one said transducer is located at the side of the conveyor path, and means are provided for producing sideways motion of the objects on the conveyor to bring them into contact with the or each transducer during passage therebeside.

8. The apparatus of claim 6, in which at least one said transducer is mounted on a paddle member positioned adjacent the conveyor and rotated at such a speed in relation tp the speed of the conveyor and the spacing of the objects thereon as to ensure an impact.

9. The apparatus of claim 1, in which said electrodes are connected to circuit means adapted to measure the resonance produced in the object by the impact.

10. The apparatus of claim 9, in which said circuit means measures the rise time or the slope of the transducer output signal.

11. The apparatus of claim 9, in which said circuit means effects a comparison of two frequency components of the transducer output signal.

*Fig. 1*

*Fig. 2*

Fig. 3

Fig. 5

VOLTS

A

B

TIME

Fig.4a

Fig.4b